# EUROPEAN PATENT APPLICATION

(11) **EP 1 815 849 A1**
(43) Date of publication of application: **08.08.2007**
(21) Application number: 06250660.5
(22) Date of filing: 08.02.2006
(51) Int. Cl.: A61K 9/26, A61K 9/16, A61K 31/55

(54) **Oxcarbazepine pharmaceutical formulation and its method of preparation, wherein oxcarbazepine has a broad and multi-modal particle size distribution**

(30) Priority: 31.01.2006 US 764134 P
(71) Applicant: Teva Pharmaceutical Industries Ltd, 49131 Petah Tiqva (IL)
(72) Inventor: Blau, Sigal, 43728 Raanana (IL); Zalit, Ilan, Rosh Ha Ayin (IL); Kolatkar, Gershon, 49275 Petah Tiqva (IL); Abrutzky Azaria, Carolina, 44448 Kfar Saba (IL)
(74) Representative: Russell, Tim

(57) **Abstract**

The present invention provides a pharmaceutical composition comprising oxcarbazepine and at least one pharmaceutical excipient, wherein the oxcarbazepine in the composition has a broad particle size distribution and an enhanced oxcarbazepine dissolution rate. The broad particle size distribution of oxcarbazepine in the pharmaceutical composition is preferably a multi-modal oxcarbazepine particle size distribution.

## Description

### RELATED APPLICATIONS

The present application claims the benefit of United States Provisional Patent Application No. 60/...,... filed January 31, 2006, which is incorporated herein by reference.

### FIELD OF THE INVENTION

The present invention relates to pharmaceutical formulations. More particularly, the present invention concerns a formulation comprising oxcarbazepine and methods for preparing this pharmaceutical formulation. Considering the very close similarity between oxcarbazepine and carbamazepine, including their similar low solubility, the present invention is equally applicable to pharmaceutical formulations comprising carbamazepine.

### BACKGROUND OF THE INVENTION

Oxcarbazepine (10-oxo-10, 11-dihydro-5H-dibenz[b, f]azepine-5-carboxamide) of the general formula: 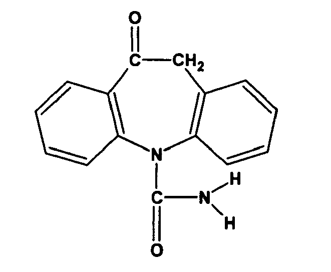 has valuable therapeutic benefits and acts as a central nervous system depressant. Currently it is being marketed as TRILEPTAL^{®}, for the treatment of epilepsy. According to the prescribing information for TRILEPTAL^{®}, the pharmacological benefit of oxcarbazepine is primarily exerted through the 10-hydroxy metabolite of oxcarbazepine. In vitro studies indicate that the metabolite blocks voltage sensitive sodium channels, which results in the stabilization of hyperexcited neural membranes, inhibition of repetitive neuronal firing, and diminution of propagation of synaptic impulses. These actions are thought to be important in the prevention of seizure spread in the brain. U.S. Pat. No. 5,658,900, incorporated herein by reference, further describes the use of oxcarbazepine to treat Parkinson's disease.

Oxcarbazepine, an antiepileptic drug, is a white to yellowish crystalline powder that is practically insoluble in water. There is a need to enhance the dissolution rate and the bioavailability of oxcarbazepine formulations. It has long been known in the art that the dissolution and bioavailability of poorly soluble drugs may be enhanced by using small particles of such drugs, and applying a narrow particle size distribution which is considered normal formulation practice. In line herewith, Schlutermann (US patent application 2003/0190361) describes a formulation comprising oxcarbazepine of fine particle size and a narrow size distribution. The particles have a median particle size of approximately 2 to 12 microns and/or leave a maximum residue on a 40 micron sieve of up to 5%. Schlutermann also describes a film-coated tablet comprising oxcarbazepine particles with the same characteristics. Further, EP0646374 describes a color stable formulation of oxcarbazepine, which is coated with two layers, wherein each layer contains white pigments.

A common method of grinding drugs employs a jet mill. The jet mill uses compressed air to produce micronized particles from large dry particles. The mill is designed in such a way that the powdered particles exit the milling chamber and are collected in a collection vessel. Waste of fine particles is also produced during the process and is collected in a filter bag. Grinding processes such as this for example are costly in both their investment, as grinding is a time consuming process, and in operating costs. In addition, such processes result in substantial losses of the drug during grinding. Furthermore, safety considerations should be taken into account during the air jet milling, due to the active pharmaceutical ingredient (API) dust formation accompanying the process. These concerns become more problematic for pharmaceutical compositions comprising oxcarbazepine due to the relatively large recommended daily dose of 1200 mg/day.

Further, Sehgal et al. (WO/2002/094774) describes a method to enhance the dissolution rate of oxcarbazepine by adding a wetting agent into the formulation. The addition of such wetting agent to the oxcarbazepine formulation, enhances the dissolution rate in-vitro.

The present invention therefore provides a process of preparing an oxcarbazepine formulation with a sufficiently high dissolution rate and good bioavailability, and which is safer and/or more economical to prepare than processes used heretofore.

### SUMMARY OF THE INVENTION

The present invention provides a pharmaceutical composition comprising, a) oxcarbazepine, and b) at least one pharmaceutical excipient, wherein the oxcarbazepine in the composition has a broad particle size distribution. The broad particle size distribution can be comprised of a multi-modal oxcarbazepine particle size distribution.

Further, there is provided a method for preparing a pharmaceutical composition of the invention comprising the steps of:
a) providing oxcarbazepine having a broad particle size distribution;
b) providing at least one excipient;
c) combining the oxcarbazepine with the at least one excipient.

Further, there is provided a method of preparing a granular composition comprising oxcarbazepine having a broad particle size distribution which comprises the following steps of
a) providing oxcarbazepine with broad particle size distribution, optionally comprising two or more populations of oxcarbazepine having different particle size distributions;
b) providing at least one excipient;
c) forming at least one granulate comprising at least one of said oxcarbazepine populations; and
d) mixing the granulated oxcarbazepine with any remaining ungranulated oxcarbazepine and one or more excipients to form a final granulate blend. When preparing tablets from the granular composition the method further comprises the steps of
e) optionally mixing the final granulate blend with one or more excipients to form a tabletting mixture;
f) pressing either the tabletting mixture or the final granulate blend into tablets; and optionally
g) coating the tablets

The present invention also provides a pharmaceutical composition comprising, a) spray granulated oxcarbazepine, and b) at least one pharmaceutical excipient.

In another aspect the present invention provides a pharmaceutical composition comprising, a) oxcarbazepine with broad particle size distribution, and b) at least one pharmaceutical excipient, optionally, the oxcarbazepine in the composition has at least two populations of different particle sizes and at least one of the populations of oxcarbazepine particles comprises spray granulated oxcarbazepine.

Further, there is provided a method of preparing a granular composition comprising at least two populations of different oxcarbazepine particle sizes wherein at least one population of oxcarbazepine particles is spray granulated comprising the following steps of
a) providing oxcarbazepine which comprises two or more populations of oxcarbazepine having different particle size distributions;
b) mixing at least one population of oxcarbazepine particles with one or more excipients and forming at least one spray granulate; and
c) mixing the at least one spray granulated oxcarbazepine population of particle sizes with any remaining non-spray granulated population of oxcarbazepine particle sizes, which together form an oxcarbazepine mixture of the total amount of oxcarbazepine in the composition. When preparing tablets from the granular composition the method further comprises at least one of the following steps d and e;
d) mixing the oxcarbazepine mixture with one or more excipients and forming a final granulate;
e) mixing the final granulate with one or more excipients to form a tabletting mixture; and then,
f) pressing the tabletting mixture into tablets; and optionally
g) coating the tablets.

In a preferred embodiment, the granulate in step c) is prepared by a spray granulation process.

Further, there is provided a method of preparing a granular composition comprising at least two populations of different oxcarbazepine particle sizes wherein at least one population of oxcarbazepine particles is spray granulated comprising the following steps of
a) providing oxcarbazepine which comprises two or more populations of oxcarbazepine having different particle size distributions; and
b) spraying at least one population of oxcarbazepine particles on a mixture of one or more excipients and any remaining population of oxcarbazepine and forming at least one spray granulate. When preparing tablets from the granular composition the method further comprises the following steps
c) optionally mixing the final granulate with one or more excipients to form a tabletting mixture;
d) pressing the tabletting mixture into tablets; and optionally
e) coating the tablets.

Further, there is provided a method of preparing a granular composition comprising at least two populations of different oxcarbazepine particle sizes wherein at least two populations of oxcarbazepine particles are spray granulated comprising the following steps of
a) providing oxcarbazepine which comprises two or more populations of oxcarbazepine having different particle size distributions;
b) spraying separately at least two populations of oxcarbazepine particles with one or more excipients and forming at least two spray granulates; and
c) mixing the at least two spray granulated oxcarbazepine populations of oxcarbazepine particle sizes. When preparing tablets from the granular composition the method further comprises at least one of the following steps
d) optionally mixing the oxcarbazepine mixture with one or more excipients and forming a final granulate;
e) mixing the final granulate with one or more excipients to form a tabletting mixture;
f) pressing the tabletting mixture into tablets; and optionally
g) coating the tablets.

Further, there is provided a method of preparing a granular composition comprising oxcarbazepine with broad particle size distribution which comprises the following steps of
a) providing oxcarbazepine which comprises broad particle size distributions; and
b) spraying the oxcarbazepine particles with one or more excipients and forming a spray granulate. When preparing tablets from the granular composition the method further comprises at least one of the following steps
c) optionally mixing the granulate with one or more excipients to form a tabletting mixture;
d) pressing the tabletting mixture into tablets; and optionally
e) coating the tablets

In another aspect the present invention also provides a pharmaceutical composition comprising oxcarbazepine with a broad particle size distribution and having a dissolution profile such that
a) no more than about 30% of the total amount of oxcarbazepine is dissolved from the composition after 35 minutes of measurement in a dissolution apparatus;
b) from about 30% to about 50%, preferably about 40% or more, of the total amount of oxcarbazepine is dissolved from the composition after 50 minutes of measurement in a dissolution apparatus; and
c) from about 30% to about 50% of the total amount of oxcarbazepine is dissolved from the composition after 60 minutes of measurement in a dissolution apparatus system that simulates the gastrointestinal environment.

In another aspect, the present invention provides pharmaceutical compositions of the invention for use in therapy, in particular for use in treating a patient suffering from epileptic seizures or Parkinson's disease or neuropathic pain. Furthermore, the present invention provides the use of oxcarbazepine for the manufacture of a pharmaceutical composition of the present invention for treating a patient suffering from epileptic seizures or Parkinson's disease or neuropathic pain.

### BRIEF DESCRIPTION OF THE DRAWINGS

**Figure 1**: Dissolution profile of oxcarbazepine tablets (prepared according to examples 1 and 2) in comparison to a TRILEPTAL^{®} bioequivalent formulation under simulated gastrointestinal conditions.
**Figure 2:** Dissolution profile of oxcarbazepine tablets (prepared according to examples 3 and 4) in comparison to a TRILEPTAL^{®} bioequivalent formulation and a non-bioequivalent formulation (prepared according to examples 7) under simulated gastrointestinal conditions.
**Figure 3:** Dissolution profile of oxcarbazepine tablets (prepared according to examples 5 and 6) in comparison to a TRILEPTAL^{®} bioequivalent formulation and a non-bioequivalent formulation (prepared according to examples 7) under simulated gastrointestinal conditions.
**Figure 4:** Dissolution profile of oxcarbazepine tablets (prepared according to examples 8 and 9) in comparison to a TRILEPTAL^{®} bioequivalent formulation under simulated gastrointestinal conditions.
**Figure 5:** Dissolution profile of oxcarbazepine tablets (prepared according to examples 10 and 11) in comparison to a TRILEPTAL^{®} bioequivalent formulation and a non-bioequivalent formulation (prepared according to examples 7) under simulated gastrointestinal conditions.
**Figure 6:** Dissolution profile of oxcarbazepine tablets (prepared according to examples 12 and 13) in comparison to a TRILEPTAL^{®} bioequivalent formulation and a non-bioequivalent formulation (prepared according to examples 7) under simulated gastrointestinal conditions.

### DETAILED DESCRIPTION OF THE INVENTION

As used herein, "broad particle size distribution" means a particular particle size distribution wherein the difference in the value of d(0.5) and d(0.95), is greater than 38 microns, and the d(0.5) value is 35 microns or less. Wherein the d(0.5) value is determined by methods such as laser diffraction and the value of d(0.95) is estimated from the sieve size through which no more than 95% of the particles would pass.

For practical reasons because there are not an infinite range of sieve sizes, the broad particle size distribution of oxcarbazepine may be characterized by having the d(0.5) ranges from 12 to 35, preferably 13 to 30 and more preferably from 14 to 25, or d(0.5) ranges from 0.01µ to 2.0µ (more preferably from 0.2 to 1.9 and most preferably from 0.4 to 1.5), while sieving of the same population using 40µ sieve results with residue of more than 5% on sieve. While the two methods of characterization are not precisely congruent they broadly define the materials of the invention, such that the few cases of a material that only matches one of the above definitions is within the contemplated scope of the invention.

The term "multi-modal particle size distribution" and "multi-modal oxcarbazepine particle size distribution" are meant to be understood as either, oxcarbazepine having a particular particle size distribution which is characterized in that a graphic plot of oxcarbazepine particle sizes by volume or weight, displays two or more peak particle sizes. Alternatively this term is understood as oxcarbazepine prepared by mixing at least two populations of oxcarbazepine characterized by different particle size distributions. A particle size distribution as described in the present invention can be determined by various conventional methods of analysis, such as Laser light scattering, laser diffraction, sedimentation methods, pulse methods, electrical zone sensing, sieve analysis and optical microscopy (usually combined with image analysis). The evaluation of particle size distribution of the multi modal particle size distribution, prepared by a mix of at least two population of oxcarbazepine, can be performed by preparing a mix of multi oxcarbazepine particle size populations at appropriate ratio and analyze their particle size distribution by laser diffraction method. Another option to evaluate the multi modal particle size distribution (PSD) is by mathematical calculation of d(0.1), d(0.5) and d(0.9) which is performed by calculation of the PSD weighted mean (average) of each of the oxcarbazepine populations.

Further, as used herein the term "unground particles" refers to particles obtained from the synthesis of oxcarbazepine (the active pharmaceutical ingredient, API) that have not been exposed to grinding or have been exposed to only minimal grinding. As used herein the term "large drug particles" refers to populations of unground drug particles, populations of particles with a median particle size of at least about 13 microns, and when sieving of the same population using 40µm sieve results with a residue of more than 5% on sieve. In contrast, as used herein the term "small drug particles" generally means populations of drug particles with sizes smaller than 6 microns, preferably smaller than 3 microns, more preferably smaller than 2 microns and while sieving of the same population using 40µm sieve results with a residue of less than 5% on sieve Also as used herein the term "spray-granulated oxcarbazepine" refers to granulated populations of oxcarbazepine particles that have been granulated by spraying an oxcarbazepine dispersion on a powder carrier.

To achieve an adequate dissolution rate of the poorly soluble active pharmaceutical ingredient oxcarbazepine from a pharmaceutical composition is an important aspect in formulating an oxcarbazepine dosage form for use in medical treatments. According to the present invention a dissolution rate similar to that of the TRILEPTAL® bioequivalent formulation is achievable with formulations having oxcarbazepine comprising a broad particle size distribution. The present invention thus provides a pharmaceutical composition comprising oxcarbazepine wherein the objectives of providing a desired dissolution rate and bioavailability using a safer method with less dust formation during the grinding process and/or reduced grinding cost and loss of active pharmaceutical ingredient are achieved by using a formulation comprising oxcarbazepine having a broad particle size distribution. One embodiment of the present invention provides a pharmaceutical composition comprising, a) oxcarbazepine, and b) at least one pharmaceutical excipient, wherein the oxcarbazepine in the composition has a broad particle size distribution. Optionally, the broad particle size distribution is a multi-modal oxcarbazepine particle size distribution.

The oxcarbazepine pharmaceutical composition of the present invention is characterized by its broad particle size distribution,. Particle size distribution can be determined by various conventional methods of analysis, such as Laser light scattering, laser diffraction, sedimentation methods, pulse methods, electrical zone sensing, sieve analysis and optical microscopy (usually combined with image analysis).

Preferably, the multi-modal oxcarbazepine particle size distribution in the pharmaceutical composition contains a multi-modal particle size distribution, i.e. at least two populations of particles. Each of these populations differ in their median particle size and have a distinct particle size distribution.

Furthermore, the pharmaceutical composition of the present invention preferably comprises unground oxcarbazepine particles as obtained from the synthesis of the active pharmaceutical ingredient (API). Unground oxcarbazepine particles may for example be characterized by d(0.1), d(0.5) and d(0.9 of about 21, 71 and 248 microns, respectively. More preferably, the multimodal oxcarbazepine pharmaceutical composition comprises at least one population of unground oxcarbazepine particles.

In a preferred embodiment of the invention, the pharmaceutical composition comprises two or more populations of oxcarbazepine particles wherein at least one of these populations is a population of large oxcarbazepine particles. A population of large oxcarbazepine particles is characterized as a population selected from the group consisting of, a population of oxcarbazepine particles with a median size of at least 13 microns, a population of unground particles, particles that do not pass a 40 micron sieve, and mixtures thereof. Preferably, a population of large oxcarbazepine particles is characterized as a population having a median oxcarbazepine particle size of at least about 13 microns. More preferably, a population of large oxcarbazepine particles is characterized as a population having a median oxcarbazepine particle size of at least about 13 microns, and the population of large oxcarbazepine particles comprises more than about 5% of particles that do not pass a 40 micron sieve.

An embodiment of the invention includes a pharmaceutical compositions of the present invention comprising large oxcarbazepine particles in an amount between about 6% by weight, and about 49% by weight of the total amount of oxcarbazepine in the pharmaceutical composition. Further, in this embodiment the pharmaceutical compositions of the present invention comprises at least one population of small oxcarbazepine particles, wherein the oxcarbazepine median particle size is smaller than about 6 microns, preferably smaller than about 3 microns, most preferably smaller than 2 micron. Such pharmaceutical composition preferably comprises small oxcarbazepine particles in an amount from about 94% by weight, to about 60% by weight of the total amount of oxcarbazepine in the pharmaceutical composition. Preferably the content of the small oxcarbazepine particles in the multi-modal pharmaceutical composition is adjusted to the content of the large oxcarbazepine particles. Thus, according to one preferred embodiment, the pharmaceutical composition comprises large oxcarbazepine particles in an amount from about 6% to about 40% by weight and small oxcarbazepine particles in an amount from about 94% to about 60% by weight of the total amount of oxcarbazepine in the pharmaceutical composition. More preferably, the pharmaceutical composition comprises large oxcarbazepine particles in an amount from about 10% to about 35% by weight and small oxcarbazepine particles in an amount from about 90% to about 65% by weight of the total amount of oxcarbazepine large particles.

Another embodiment of a pharmaceutical compositions of the present invention comprise large oxcarbazepine particles in an amount of between about 95% by weight, and about 51% by weight of the total amount of oxcarbazepine in the pharmaceutical composition. Further, the pharmaceutical compositions of this embodiment present invention comprising at least one population of small oxcarbazepine particles, wherein the oxcarbazepine particle size is smaller than about 6 microns, preferably smaller than about 3 microns, most preferably smaller than 2 micron. Furthermore, such pharmaceutical compositions comprises small oxcarbazepine particles in an amount from about 5% to about 49% by weight, Preferably the content of the small oxcarbazepine particles in the pharmaceutical composition is adjusted to the content of the large oxcarbazepine particles. Thus, according to one preferred embodiment, the pharmaceutical composition comprises large oxcarbazepine particles in an amount from about 90% to about 60% by weight and small oxcarbazepine particles in an amount from about 10% to about 40% by weight of the total amount of oxcarbazepine in the pharmaceutical composition. More preferably, the pharmaceutical composition comprises large oxcarbazepine particle in an amount from about 85% to about 65% by weight and small oxcarbazepine particles in an amount from about 15% to about 35% by weight of the total amount of oxcarbazepine large particles.

In another embodiment of the present invention, the oxcarbazepine has a median particle size of not more than 2 microns and has a minimum residue on a 40 micron sieve of more than 5%.

In another embodiment of the present invention, the oxcarbazepine has a median particle size of not less than 13 microns and has a minimum residue on a 40 micron sieve of more than 5%.

In another embodiment of the present invention, the oxcarbazepine has a median particle size of 2 to 12 microns and has a minimum residue on a 40 micron sieve of more than 5%.

Another embodiment of the present invention provides a pharmaceutical composition comprising, a) oxcarbazepine, and b) at least one pharmaceutical excipient, wherein the oxcarbazepine in the composition has a particle size distribution characterized by oxcarbazepine with d(0.5) ranges from 13 to 40 microns and more than 5% residue on 40 micron sieve. The oxcarbazepine can be from a single population of particles.

The oxcarbazepine pharmaceutical compositions comprising a broad particle size distribution as in the present invention further may contain excipients such as tablet and capsule fillers and diluents (such as microcrystalline cellulose, lactose, starch and tri-basic calcium phosphate), disintegrants (such as starch, croscarmellose sodium and sodium starch glycolate), binders (such as starch, hydroxypropyl methyl cellulose and Povidone), glidant (such as colloidal silicon dioxide), lubricants (such as magnesium stearate, magnesium lauryl sulfate and sodium stearyl fumarate) and surfactants and wetting agents (such as sodium lauryl sulfate, polysorbate and poloxamer).

More particularly, suitable diluents and fillers for use in the pharmaceutical composition of the present invention include microcrystalline cellulose (e.g. Avicel^{®}), microfine cellulose, lactose, starch, pregelatinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, potassium chloride, powdered cellulose, sodium chloride, sorbitol and talc.

Further, suitable surfactants for use in the pharmaceutical composition of the invention include poloxamers, polyethylene glycols, polysorbates, sodium lauryl sulfate, polyethoxylated castor oil, and hydroxylated castor oil.

Solid pharmaceutical compositions that are compacted into a dosage form, such as a tablet, may include excipients whose functions include helping to bind the active ingredient and other excipients together after compression. Binders for solid pharmaceutical compositions include acacia, alginic acid, carbomer (e.g. carbopol), carboxymethylcellulose sodium, dextrin, ethyl cellulose, gelatin, guar gum, hydroxyethyl cellulose, hydroxypropyl cellulose (e.g. Klucel^{®}), hydroxypropyl methyl cellulose (e.g. Methocel^{®}), liquid glucose, maltodextrin, methylcellulose, polymethacrylates, povidone (e.g. Povidone PVP K-30, Kollidon^{®}, Plasdone^{®}), pregelatinized starch, sodium alginate and starch.

A compacted solid pharmaceutical composition may also include the addition of a disintegrant to the composition. Disintegrants include croscarmellose sodium (e.g. Ac Di Sol^{®}, Primellose^{®}), crospovidone (e.g. Kollidon^{®}, Polyplasdone^{®}), microcrystalline cellulose, polacrilin potassium, powdered cellulose, pregelatinized starch, sodium starch glycolate (e.g. Explotab^{®}, Primoljel^{®}) and starch.

Glidants can be added to improve the flowability of a non compacted solid composition and to improve the accuracy of dosing. Excipients that may function as glidants include colloidal silicon dioxide, magnesium trisilicate, powdered cellulose, and talc.

A lubricant can be added to the composition to reduce adhesion and/or ease the release of the product from e.g. the dye. Lubricants include magnesium stearate, calcium stearate, glyceryl monostearate, glyceryl palmitostearate, hydrogenated castor oil, hydrogenated vegetable oil, mineral oil, polyethylene glycol, sodium lauryl sulfate, sodium stearyl fumarate, stearic acid, talc and zinc stearate.

Other excipients that may be incorporated into the formulation include preservatives, antioxidants, or any other excipient commonly used in the pharmaceutical industry.

The solid compositions of the present invention include powders, granulates, aggregates and compacted compositions. The dosages include dosages suitable for oral, buccal, and rectal administration. Although the most suitable administration in any given case will depend on the nature and severity of the condition being treated, the most preferred route of the present invention is oral. The dosages may be conveniently presented in unit dosage form and prepared by any of the methods well known in the pharmaceutical arts.

The present invention also discloses a method for the production of oxcarbazepine pharmaceutical composition with broad particle size distribution. e.g. one of the methods comprises a first step of mixing large oxcarbazepine particles with small oxcarbazepine particles. This mixing can be carried out either prior to granulation or during granulation. Alternatively, at least part of the large size oxcarbazepine particles, for example at least one population of large oxcarbazepine particles, and at least part of the small size oxcarbazepine particles, for example at least one population of small oxcarbazepine particles, are granulated separately and the formed granules are mixed. The mixture can then be combined with additional excipients and pressed into tablets.

According to a preferred embodiment, the process involves milling and optionally de-agglomerating a portion of the total amount of oxcarbazepine to form small drug particles. Preferably, the remaining amount of oxcarbazepine is not milled and used as such forming a population of large drug particles. Alternatively, this remaining amount of oxcarbazepine is only slightly milled forming large drug particles of the multi-modal pharmaceutical composition of the present invention. Various known methods can be used for milling and de-agglomeration of oxcarbazepine particles. A jet mill, impact mill, ball mill, vibration mill, mortar mill or pin mill may for example be used for milling unground oxcarbazepine. Preferably, the oxcarbazepine is milled in a liquid dispersion by a homogenizer, such as rotor-stator or high pressure homogenizer such as a microfluidizer®, which produces milled oxcarbazepine at a high yield. Preferably, such reduction of the particle size of oxcarbazepine by wet milling is carried out in the presence of a hydrophilic polymer or stabilizer. A preferred hydrophilic polymer or stabilizer is a hypromellose (Hydroxypropylmethylcellulose, HPMC), for example Pharmacoat®.

Preferably, the method of the present invention produces compressed solid dosage forms. There are three well known processes for manufacturing such dosage forms; (i) direct compression, (ii) dry granulation and (iii) wet granulation. There are two well known processes for wet granulation. A wet granulate can be prepared using a mixer and subsequently the wet granulate is dried in order to obtain a dry homogenous granulate. In another method a wet granulate is prepared by spray granulation. In a fluid-bed, spray granulation process, particles and granulate are built up in a fluid bed by spraying a liquid onto fluidized particles. Thus in such process materials are fluidized in the fluid bed dryer and subsequently a solution is sprayed through a nozzle.

The pharmaceutical composition of the present invention may be prepared in any dosage form such as a compressed granulate in the form of a tablet for example. Also, uncompressed granulates and powder mixes that are obtained by the method of the present invention in the precompression steps can be simply provided in dosage form of a capsule or sachet. Therefore, dosage forms of pharmaceutical composition of the present invention include solid dosage forms like tablets, powders, capsules, sachets, troches and lozenges. The dosage form of the present invention may also be a capsule containing the composition, preferably a powdered or granulated solid composition of the invention, within either a hard or soft shell. The shell may be made from gelatin and optionally contain a plasticizer such as glycerin and sorbitol, and an opacifying agent or colorant.

Preferably, the production of the pharmaceutical composition comprising a broad particle size distribution of oxcarbazepine comprises spray granulation of at least one population of oxcarbazepine particles, either from small drug particles or from large drug particles. In another preferred embodiment of the preparation method, the process comprises spray granulation of both a population of small and large drug particles together. In this later embodiment, a suitable dissolution rate and bioavailability obtained at reduced grinding cost, higher safety and less loss of active pharmaceutical ingredient are achieved by using pharmaceutical compositions comprising spray-granulated oxcarbazepine. Spray-granulated particles are described in various publications, e.g. on the website of Glatt, a manufacturer of spray granulation equipment. Preferably, spray-granulated oxcarbazepine is a product of spraying a dispersion of oxcarbazepine particles on air-fluidized excipient particles.

In one embodiment, the sprayed oxcarbazepine dispersion comprises relatively large oxcarbazepine particles. These relatively large particles comprise oxcarbazepine particles as are obtained from synthesis without being milled, for example particles characterized with d(0.1), d(0.5) and d(0.9) of about 21, 71 and 248 microns, respectively, or those which have been subjected to only minimal grinding. Relatively large particles for use in spray granulation may also be described as particles with a median size greater than 13 microns, or oxcarbazepine particles that do not pass a 40 micron sieve. Optionally, the pharmaceutical composition of spray granulated oxcarbazepine also comprises oxcarbazepine particles of smaller size which are spray granulated. Optionally, the oxcarbazepine dispersion for use in spray granulation has a multimodal oxcarbazepine particle size distribution similar to the oxcarbazepine in a multi-modal oxcarbazepine pharmaceutical composition.

In another embodiment, the sprayed oxcarbazepine dispersion comprises relatively small oxcarbazepine particles. These relatively small particles comprise oxcarbazepine particles as are obtained following grinding process. Relatively small particles for use in spray granulation may also be described as particles with a median size smaller than 6 microns, preferably smaller than 3 microns. Optionally, the pharmaceutical composition of spray granulated oxcarbazepine also comprises oxcarbazepine particles of larger size which are spray granulated. Optionally, the oxcarbazepine dispersion for use in spray granulation has a multi-modal oxcarbazepine particle size distribution similar to the oxcarbazepine in a multi-modal oxcarbazepine pharmaceutical composition.

The pharmaceutical compositions of spray-granulated oxcarbazepine comprise at least one excipient selected from the group such as tablet and capsule fillers and diluents (such as microcrystalline cellulose, lactose, starch and tri-basic calcium phosphate), disintegrants (such as starch, croscarmellose sodium and sodium starch glycolate), binders (such as starch, hydroxypropyl methyl cellulose and Povidone), glidant (such as colloidal silicon dioxide), lubricants (such as magnesium stearate, magnesium lauryl sulfate and sodium stearyl fumarate) and surfactants and wetting agents (such as sodium lauryl sulfate, polysorbate and poloxamer). In a preferred embodiment, the sprayed dispersion of oxcarbazepine comprises a binder, such as hypromellose.

The present invention also discloses a method for the production of a pharmaceutical composition of spray-granulated oxcarbazepine. The method involves spraying of at least one excipient with a dispersion of oxcarbazepine. First a dispersion of oxcarbazepine is prepared and this dispersion of oxcarbazepine is sprayed onto fluidized excipients particles, which are then dried, preferably rapidly in the fluidizing/ suspending gas (typically, air). The spraying process is preferably done by Fluidized bed technology equipment, where the particles are suspended in a vertical column with a rising air stream. While the particles are fluidized, the coating dispersion of oxcarbazepine is sprayed into the column. This spraying can be carried out by any one of three methods; top spray, bottom spray and a "tangential" or powder spray. Preferably the spraying of the oxcarbazepine dispersion is carried out by a top spray method.

Moreover, the preferred spray-granulation method of the present invention differs somewhat from the commonly used spray-granulation method. In the commonly used procedure a granulation solution is sprayed onto a mixture of active and inactive materials which are suspended or fluidized in air. In the preferred method of spray-granulation of the present invention, a dispersion of the active material is sprayed on excipients suspended in air or on a mixture of active and inactive materials.

In particular, when in the above spray granulation method two separate granulations are prepared, one comprising small oxcarbazepine particles and the other comprising large oxcarbazepine particles, a further advantage of the invention is achieved. It is often important to adjust and fine tune the dissolution characteristics of the final pharmaceutical compositions during development. Once the dissolution rate is determined for each granulation, such fine tuning of the dissolution rate for the final pharmaceutical composition becomes extremely simple. A mere mixing of the two or more granulates in appropriate proportions will achieve the desired resulting dissolution rate. When a higher rate of dissolution is required, the mixture of granulates will comprise a proportionately higher amount of the faster dissolving granulates. The reverse is similarly simple.

The pharmaceutical compositions of the present invention provide a dissolution rate for oxcarbazepine that is similar to a pharmaceutical composition comprising ground oxcarbazepine particles. This is in contrast to the expected dissolution rate for oxcarbazepine particles having a broad particle size distribution, because the pharmaceutical composition of the present invention comprises large/unground oxcarbazepine particles. Furthermore, the dissolution rate of oxcarbazepine from the pharmaceutical composition of the present invention is sufficient for gastro-intestinal absorption of oxcarbazepine in the slightly acidic and neutral pH region. Oxcarbazepine dissolves from the pharmaceutical composition of the present invention at a suitable rate. Preferably, at least 30% of the oxcarbazepine in the pharmaceutical composition dissolves from the composition in a simulated gastro-intestinal environment within 60 minutes. More preferably, at least 30% or more, preferably about 40% or more, of the oxcarbazepine in the pharmaceutical composition dissolves from the composition in such environment within 50 minutes, and most preferably at least 20% of the oxcarbazepine is dissolved from the pharmaceutical composition in such environment within 35 minutes. The preferred dissolution rate for oxcarbazepine from the pharmaceutical composition of the present invention in a simulated gastro-intestinal environment may also be described as; a) no more than about 30% of the total amount of oxcarbazepine is dissolved from the composition after 35 minutes of measurement in a dissolution apparatus; b) from about 30%, or preferably about 40% or more, to about 50% of the total amount of oxcarbazepine is dissolved from the composition after 50 minutes of measurement in a dissolution apparatus; and c) from about 30% to about 50% of the total amount of oxcarbazepine is dissolved from the composition after 60 minutes of measurement in a dissolution apparatus.

Oxcarbazepine is used for the treatment of epilepsy in patients suffering from epileptic seizures. The pharmaceutical compositions of the present invention provide an effective delivery system for the administration of oxcarbazepine to patients in need of such treatment. Treatment of patients suffering from epilepsy may comprise administering an effective amount of oxcarbazepine in a pharmaceutical composition of the present invention. Preferably, the pharmaceutical composition comprises a broad particle size distribution of oxcarbazepine, more preferably the broad oxcarbazepine particle size distribution is a multi-modal oxcarbazepine particle size distribution. In addition, oxcarbazepine has been shown to be effective to treat Parkinson's disease. The pharmaceutical composition of the present invention therefore also provides an effective delivery system for the administration of oxcarbazepine to patients suffering from Parkinson's disease and neuropathic pains.

The following examples are presented in order to further illustrate the invention. These Examples should not be construed in any manner to limit the invention.

### EXAMPLES

Formulations comprising oxcarbazepine with broad particle size distribution and at least one pharmaceutical excipient were prepared. Some of the examples below demonstrate the perpetration of multi-modal tablet formulations. In order to evaluate the median oxcarbazepine particle size distribution of the multi-modal formulation two optional strategies were preformed; 1) multi-modal oxcarbazepine mixtures were prepared and their particle size distribution was analyzed using laser diffraction method (Malvern Mastersizer S); or 2) mathematical calculation of d(0.1), d(0.5) and (d(0.9) by the calculation of the PSD weighted mean of the large and small oxcarbazepine. The following table describes for example a mathematical calculation of PSD of a multi-modal mixture containing small and large oxcarbazepine particles at a 35:65 ratio, respectively.

**Table 1: Calculation of particle size distribution containing small and large particles**

| **Size (micron)** | **Volume under %** | **35%** | **Size (micron)** | **Volume under%** | **65%** | **Particle Size (Micron)** | **Volume under %** |
|---|---|---|---|---|---|---|---|
| 0.2 | 7.0 | 2.5 | 0.2 | 0.1 | 0.0 | 0.2 | 2.5 |
| 0.5 | 40.0 | 14.0 | 0.5 | 1.0 | 0.7 | 0.5 | 14.7 |
| 2.0 | 71.7 | 25.1 | 2.0 | 4.1 | 2.7 | 2.0 | 27.8 |
| 4.2 | 83.1 | 29.1 | 4.2 | 8.7 | 5.6 | 4.2 | 34.7 |
| 4.9 | 85.2 | 29.8 | 4.9 | 10.3 | 6.7 | 4.9 | 36.5 |
| 12.2 | 95.4 | 33.4 | 12.2 | 25.1 | 16.3 | 12.2 | 49.7 |
| 14.2 | 96.5 | 33.8 | 14.2 | 28.8 | 18.7 | 14.2 | 52.5 |
| 26.2 | 99.5 | 34.8 | 26.2 | 48.9 | 31.8 | 26.2 | 66.6 |
| 30.5 | 99.8 | 34.9 | 30.5 | 55.3 | 35.9 | 30.5 | 70.9 |
| 48.3 | 100.0 | 35.0 | 48.3 | 75.7 | 49.2 | 48.3 | 84.2 |
| 190.8 | 100.0 | 35.0 | 190.8 | 99.8 | 64.8 | 190.8 | 99.8 |
| 222.3 | 100.0 | 35.0 | 222.3 | 100.0 | 65.0 | 222.3 | 100.0 |

### Calculation stages:

1. Data: volume under % of the small and large oxcarbazepine.
2. Each volume under % is multiple by fraction % (35% or 65% in this example).
3. Sum of 35% product + 65% product

### Example 1: Preparation of multi-modal oxcarbazepine formulation with a 10:90 ratio.

In a first step 1 a preparation of a start dispersion from large Oxcarbazepine was prepared. 1200g of large Oxcarbazepine raw material (RM) [d(0.9)=248.5] was dispersed in a solution of 240g hypromellose (Pharmacoat 603) in 4800g of purified water. The Pharmacoat was added to the water and mixed until a clear mixture was obtained. Subsequently, the large Oxcarbazepine was added into the Pharmacoat water mixture and dispersed using a rotor stator (Brogtec) for about 30 min. The large Oxcarbazepine particle size distribution, as measured by the laser diffraction method (Malvern Mastersizer S), was as follows:

| Oxcarbazepine | d(0.1) | d(0.5) | d(0.9) |
|---|---|---|---|
| Large Oxcarbazepine | 25.1 | 86.1 | 308.1 |

In a second step 2 the particle size of the large oxcarbazepine was reduced by a high pressure homogenization process (MFIC microfluidizer M-110F). This particle size reduction by wet milling was carried out in the presence of a hydrophilic polymer or stabilizer as hypromellose (Hydroxy propyl Methyl Cellulose, for example Pharmacoat®). The particle size distribution of a final Oxcarbazepine dispersion (small Oxcarbazepine), as measured by the laser diffraction method (Malvern Mastersizer S), was as follows:

| Oxcarbazepine | d(0.1) | d(0.5) | d(0.9) |
|---|---|---|---|
| small oxcarbazepine | 0.3 | 0.6 | 2.2 |

In a third step 3 the small Oxcarbazepine final dispersion was used as spray granulation dispersion in fluid-bed-top-spray-granulation process. The granulate formulation contains the following substances:

| Batch# 1 | Amount/dose |
|---|---|
| Oxcarbazepine [as sprayed dispersion (from step 2)] | 600 mg |
| Pharmacoat [as sprayed dispersion (from step 2)] | 120 mg |
| Avicel PH 101 | 200 mg |
| Lactose monohydrate 100 mesh | 140 mg |
| Starch 1500 NF | 90 mg |
| Sodium starch glycolate | 50 mg |

In a fourth step 4 a granulate of large Oxcarbazepine was prepared in a high sheer mixer. The formulation of large oxcarbazepine granulate was as follows:

| Batch # 2 | Amount/dose |
|---|---|
| **Part 1** | |
| large oxcarbazepine [d(0.9)=248.5] | 600 mg |
| Avicel PH 101 | 120 mg |
| Pharmacoat 603 in aqueous granulation solution | 16 mg |
| Purified water | q.s. |
| **Part 2** | |
| Avicel PH 102 | 14 mg |
| Crosspovidone | 38 mg |
| Aerosil 200 | 4 mg |
| Total weight | 792 mg |

The particle size distribution of large Oxcarbazepine RM used for the granulate, as measured by laser diffraction method (Malvern mastersizer S), was as follows:

| Oxcarbazepine | d(0.1) | d(0.5) | d(0.9) |
|---|---|---|---|
| Large Oxcarbazepine (2) | 20.9 | 71.5 | 248.5 |

The final fifth step 5 involved the preparation of granulate mixture and tablets. The sprayed granulate of step 3 was mixed together with the granulate of step 4 in a 9:1 ratio, respectively. In preparing a 600mg dose a mixture of the two granulates was prepared, wherein the sprayed granulate contained 540mg of active material and the granulate of step 4 contained 60mg of active material. Consequently the final formulation, including a lubricant as an additional excipient, was as follows.

| Formulation of example 1(Batch #3) | Amount/dose |
|---|---|
| Large Oxcarbazepine granulate (batch #2) | 79 mg |
| Small Oxcarbazepine granulate (batch #1) | 1086 mg (after potentcy correction) |
| Magnesium stearate | 15 mg |
| Total weight | 1180 mg |

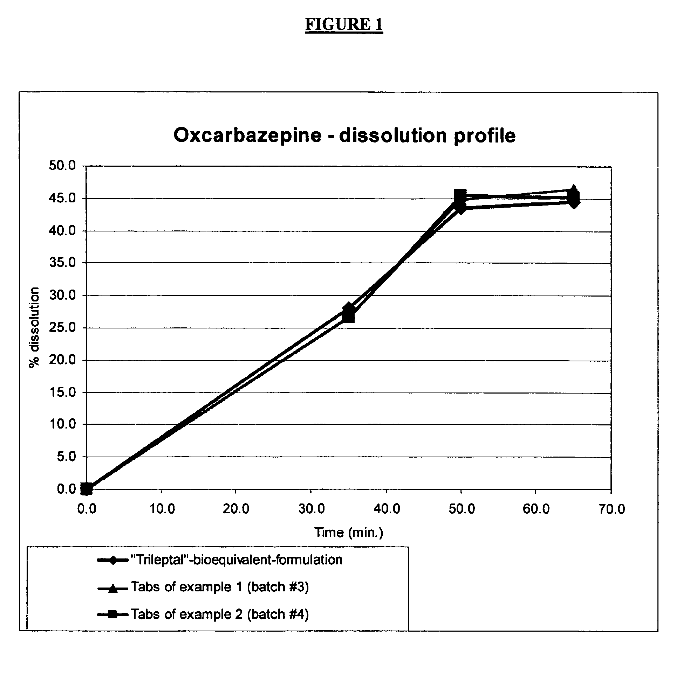

The granulate mixture was subsequently pressed into tablets and a dissolution was performed. The observed dissolution rate was similar to that for the TRILEPTAL® bioequivalent formulation as is shown in figure 1.

The particle size of the oxcarbazepine used in this example was estimated to be such that d(0.5) is of the order of 0.6 microns and about 6% of this material would be retained on a 40 micron sieve. The following exemplifies how such estimation is measured. In order to evaluate the median particle size of a multi-modal oxcarbazepine formulation with a 10%90 ratio, a mix of two dispersions was preformed. The mix contains 90% of small particles characterized by d(0.5) of 0.7 micron and 10% of large particle size characterized by d(0.5) of 68 microns. The median particle size of the mix as measured by malvern was 0.7 microns. In order to evaluate the amount of oxcarbazepine retained on a 40 micron screen, sieve analysis of the large oxcarbazepine was preformed by alpine. The result of the sieve analysis demonstrates that about 60% of the oxcarbazepine is retained on the 40 micron sieve. Therefore, a mix of 90/10 will retain at least 6% on a 40 micron sieve, assuming that no powder would be retained on the 40 micron mesh screen after sieving of the "small" oxcarbazepine particles.

### Example 2: Preparation of multi-modal oxcarbazepine formulation with a 1:5 ratio.

The first four steps were as in example 1. In the final step 5 of preparing a granulate mixture and tablets the sprayed granulate was mixed together with the granulate of step 4 in a 16.6:83.3 ratio, respectively. In preparing a 600mg dose a mixture of the two granulates was prepared, wherein the sprayed granulate contains 500mg of active material and the mixed-granulate contains 100mg of active material. Consequently the final formulation, including a lubricant as an additional excipient, was as follows.

| **Formulations of example 2 (batch # 4)** | **Amount/dose** |
|---|---|
| Large Oxcarbazepine granulate (batch #2) | 132 mg |
| Small Oxcarbazepine granulate (batch #1) | 1005 mg |
| Magnesium stearate | 15 mg |
| Total weight | 1152 mg |

The granulate mixture was subsequently pressed into tablets and a dissolution was performed. The observed dissolution rate was similar to that for the TRILEPTAL® bioequivalent formulation as is shown in figure 1. The particle size of the oxcarbazepine used in this example was estimated to be such that d(0.5) is of the order of 0.7 microns and about 10% of this material would be retained on a 40 micron sieve. The following exemplifies how such estimation is measured. In order to evaluate the median particle size of a multi-modal oxcarbazepine formulation with a 1:5 ratio, a mix of two dispersions at a close ratio was preformed. The mix contains 80% of small particles characterized by d(0.5) of 0.7 micron and 20% of large particle size characterized by d(0.5) of 68 microns. The median particle size of the mix as measured by malvern was 0.8 micron. Thus, the median particle size of 1:5 ratio is even less than 0.8 micron. In order to evaluate the amount of oxcarbazepine retained on 40 micron sieve, analysis of large oxcarbazepine was preformed by alpine. The result of the sieve analysis demonstrates that about 60% of the oxcarbazepine is retained on the sieve. Therefore, a mix of 1:5 will retain about 10% on a 40 micron sieve, assuming that no powder would be retained on 40 micron screen after sieving of the small oxcarbazepine particles.

### Example 3: Preparation of multi-modal oxcarbazepine formulation with a 55:45 ratio using a combination of a sprayed granulate and mixed granulate.

The first three steps were as in example 1. In step 4 a granulate of Oxcarbazepine [d(0.5)=30.5] was prepared by a wet granulation process. The formulation of large oxcarbazepine granulate was as follows:

| Batch # 5 | Amount/dose |
|---|---|
| **Part 1** | |
| Large oxcarbazepine [d(0.5)=30.5] | 600 mg |
| Avicel PH 101 | 120 mg |
| Pharmacoat 603 in aqueous granulation solution | 16 mg |
| Purified water | q.s. |
| **Part 2** | |
| Avicel PH 102 | 14 mg |
| Crosspovidone | 38 mg |
| Aerosil 200 | 4 mg |
| **Part 3** | |
| Magnesium stearate | 8 mg |
| Total weight | 800 mg |

The particle size distribution of large Oxcarbazepine RM used for the granulate, as measured by laser diffraction method (Malvern mastersizer S), was as follows:

| Oxcarbazepine | d(0.1) | d(0.5) | d(0.9) |
|---|---|---|---|
| Large Oxcarbazepine | 4.0 | 30.5 | 62.5 |

The final fifth step 5 involved the preparation of granulate mixture and tablets. The sprayed granulate of step 3 was mixed together with the mixed-granulate of step 4 in a 45:55 ratio, respectively. In preparing a 600mg dose a mixture of the two granulates was prepared, wherein the sprayed granulate contains 270mg of active material and the mixed-granulate from step 4 contains 330mg of active material. Consequently the final formulation, including a lubricant as an additional excipient, was as follows.

| **Formulation of example 3 (batch #6)** | **Amount/dose** |
|---|---|
| Large Oxcarbazepine granulate (batch #5) | 440 mg |
| Small Oxcarbazepine granulate (batch #1) | 543 mg (after potentcy correction) |
| Magnesium stearate | 10 mg |
| Total weight | 993 mg |

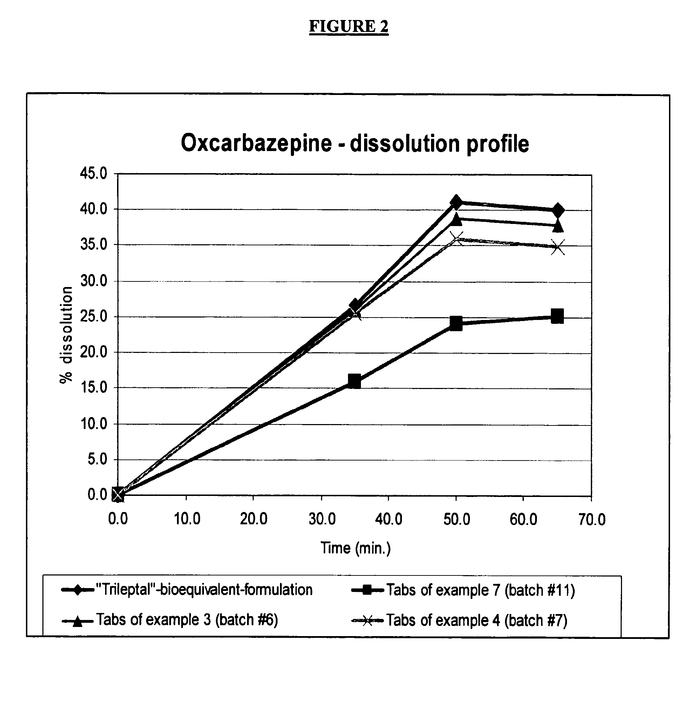

The granulate mixture was subsequently pressed into tablets and a dissolution test was performed. The observed dissolution rate was similar to that for the TRILEPTAL® bioequivalent formulation as is shown in figure 2.

### Example 4: Preparation of multi-modal oxcarbazepine formulation with a 65:35 ratio using a combination of a sprayed granulate and mixed granulate.

The first four steps were as in example 3. In the final step 5 of preparing a granulate mixture and tablets the sprayed granulate was mixed together with the mixed-granulate in a 35:65 ratio, respectively. In preparing a 600mg dose a mixture of the two granulates was prepared, wherein the sprayed granulate (small) contains 210mg of active material and the mixed-granulate (large) from step 4 contains 390mg of active material. Consequently the final formulation, including a lubricant as an additional excipient, was as follows.

| **Formulation of example 4 (batch #7)** | **Amount/dose** |
|---|---|
| Large Oxcarbazepine granulate (batch #5) | 520 mg |
| Small Oxcarbazepine granulate (batch #1) | 422 mg * (after potentcy correction) |
| Magnesium stearate | 10 mg |
| Total weight | 952 mg |

| | |
|---|---|
| * assayed for oxcarbazepine potency to comprise 210 mg oxcarbazepine | |

The granulate mixture was subsequently pressed into tablets and a dissolution test was performed. The observed dissolution rate was similar to that for the TRILEPTAL® bioequivalent formulation as is shown in figure 2.
In order to evaluate the median particle size of the 65:35 mixture contains 65% population of d(0.5) = 27.6 micron and 35% population of d(0.5)=0.8, a mathematical calculation was preformed. The calculation result indicates that the median particle size of that mixture is about 13 microns.

### Example 5: Preparation of multi-modal oxcarbazepine formulation with a 65:35 ratio using a combination of two spray granulates.

The first three steps were as in example 1. In step 4 a dispersion for spray granulation is prepared. 60g of Large Oxcarbazepine raw material (RM) [d(0.9)=248.5] was dispersed in a solution of 12g hypromellose (Pharmacoat 603) in 240g of purified water. The Pharmacoat was added to the water until a clear mixture was obtained. Subsequently, the Large Oxcarbazepine was added into the Pharmacoat water mixture and dispersed using a rotor stator (Brogtec) for about 30 min forming a large oxcarbazepine dispersion. The large Oxcarbazepine particle size distribution following the rotor stator process, as measured by the laser diffraction method (Malvern Mastersizer S), was as follows:

| Oxcarbazepine | d(0.1) | d(0.5) | d(0.9) |
|---|---|---|---|
| Large Oxcarbazepine | 3.3 | 27.6 | 90.4 |

In step 5 the large Oxcarbazepine dispersion was used as spray granulation dispersion in a fluid-bed-top-spray-granulation process. The granulate formulation contains the following substances:

| **Batch #8** | **Amount/dose** |
|---|---|
| Oxcarbazepine [as sprayed dispersion (from step 4)] | 600 mg |
| Pharmacoat [as sprayed dispersion (from step 4)] | 120 mg |
| Avicel PH 101 | 250 mg |
| Lactose monohydrate 100 mesh | 100 mg |
| Starch 1500 NF | 100 mg |
| Sodium starch glycolate | 75 mg |

The final sixth step 6 involved the preparation of granulate mixture and tablets. The sprayed granulate of step 3 was mixed together with the sprayed-granulate of step 5 in a 35:65 ratio, respectively. In preparing a 600mg dose a mixture of the two granulates was prepared,
wherein the sprayed granulate contains 210mg of active material and the sprayed-granulate from step 5 contains 390mg of active material. Consequently the final formulation, including a lubricant as an additional excipient, was as follows.

| **Formulations of example 5 (batch #9)** | **Amount/dose** |
|---|---|
| Large Oxcarbazepine granulate (batch #8) | 836 mg |
| Small Oxcarbazepine granulate (batch #1) | 422 mg |
| Magnesium stearate | 10 mg |
| Total weight | 1268mg |

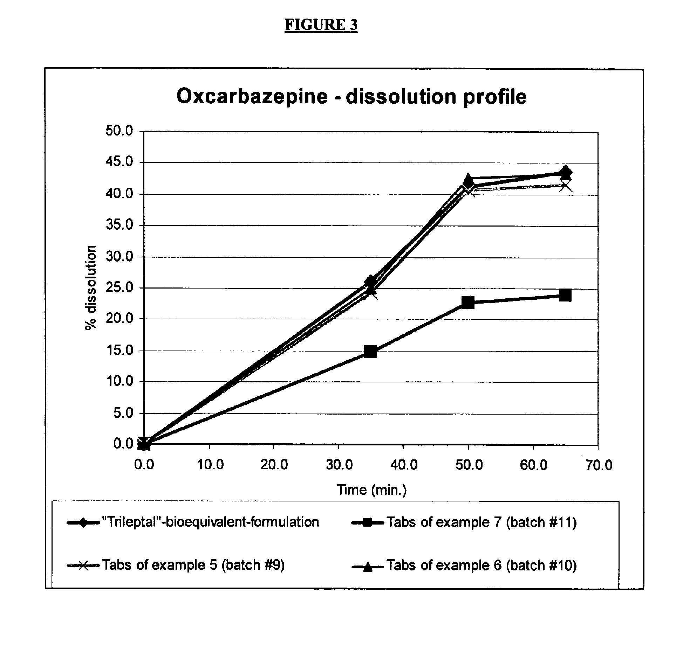

The granulate mixture was subsequently pressed into tablets and a dissolution test was performed. The observed dissolution rate was similar to that for the TRILEPTAL® bioequivalent formulation as is shown in figure 3.
In order to evaluate the median particle size of the 65:35 mixture, a mathematical calculation was preformed. The results of the calculation result indicates that the median particle size of the mixture is about 13 microns.

### Example 6: Preparation of multi-modal oxcarbazepine formulation with a 55:45 ratio using a combination of two spray granulates.

The first five steps were as in example 5. In the final step 6 of preparing a granulate mixture and tablets the sprayed-granulate of step 3 was mixed together with the sprayed-granulate of step 5 in a 45:5 ratio, respectively. In preparing a 600mg dose a mixture of the two granulates was prepared, wherein the sprayed granulate of step 3 contains 270mg of active material and the sprayed-granulate from step 5 contains 330mg of active material. Consequently the final formulation, including a lubricant as an additional excipient, was as follows.

| **Formulation of example 6 (batch#10)** | **Amount/dose** |
|---|---|
| Large Oxcarbazepine granulate (batch #8) | 707 mg |
| Small Oxcarbazepine granulate (batch #1) | 543 mg |
| Magnesium stearate | 10 mg |
| Total weight | 1260 mg |

The granulate mixture was subsequently pressed into tablets and a dissolution test was performed. The observed dissolution rate was similar to that for the TRILEPTAL® bioequivalent formulation as is shown in figure 3.

### Example 7: Preparation of relatively slow dissolving pharmaceutical composition.

A non- bioequivalent formulation with relatively slow dissolving pharmaceutical composition was prepared with oxcarbazepine characterized by d(0.5) of 43 microns. The oxcarbazepine granulate was prepared using high sheer mixer and dried by fluid bed drier. The final formulation, including a lubricant as an additional excipient, was as follows.

| Formulations of example (batch # 11) | Amount/dose |
|---|---|
| **Part 1** | |
| Oxcarbazepine | 600 mg |
| Lactose monohydrate NF 200 mesh | 100 mg |
| PVP K-30 | 16 mg |
| Purified water | q.s. |
| **Part 2** | |
| Sodium starch glycolate NF | 10 |
| Avicel PH 102 (microcrystaline cellulose) | 34 |
| Aerosil 200 (colloidal silicone dioxide) | 4 |
| **Part 3** | |
| Magnesium stearate | 8 |
| Total Weight | 800 |

The final blend was compressed into tablets and the tablets were coated.

### Example 8: Preparation of sprayed granulated oxcarbazepine formulation.

In a first step, a preparation of start dispersion from large Oxcarbazepine was prepared. 60g of Large Oxcarbazepine raw material (RM) [d(0.9)=248.5] was dispersed in a solution of 12g hypromellose (Pharmacoat 603) in 240g of purified water. The Pharmacoat was added to the water and mixed until clear mixture was obtained. Subsequently, the Large Oxcarbazepine was added into the Pharmacoat water mixture and dispersed using a rotor stator (Brogtec) for 30 min at 9000 rpm. The Oxcarbazepine particle size distribution following rotor stator process, as measured by the laser diffraction method (Malvern Mastersizer S), was as follows:

| | d(0.1) | d(0.5) | d(0.9) |
|---|---|---|---|
| Large Oxcarbazepine | 5 microns | 27 microns | 76 microns |

In a second step, the large Oxcarbazepine dispersion was used as spray granulation dispersion in fluid-bed-top-spray-granulation process. The granulate formulation contains the following substances:

| **Formulations of example 8 (Batch # 12)** | **Amount/dose** |
|---|---|
| Oxcarbazepine [as sprayed dispersion (from step 1)] | 600 mg |
| Pharmacoat [as sprayed dispersion (from step 1)] | 120 mg |
| Avicel PH 101 | 250 mg |
| Lactose monohydrate 100 mesh | 250 mg |
| Starch 1500 NF | 100 mg |
| Sodium starch glycolate | 75 mg |

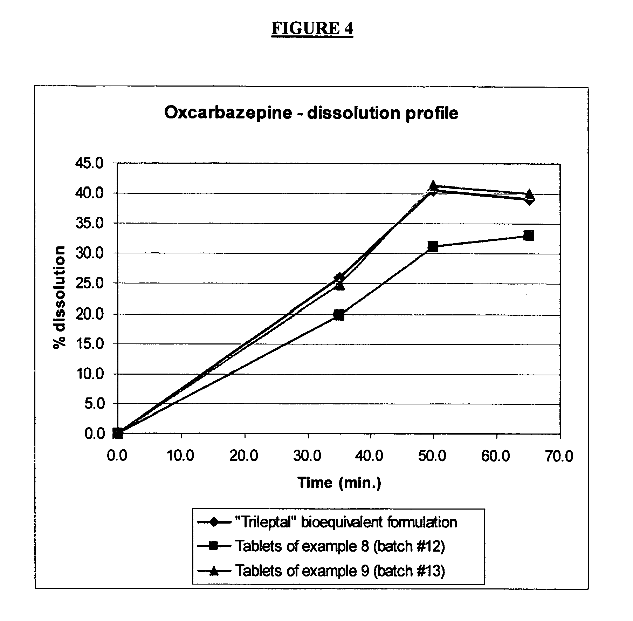

In the third step The granulate mixture was subsequently pressed into tablets and a dissolution test was performed. The observed dissolution rate was almost similar to that for the TRILEPTAL® bioequivalent formulation as is shown in figure 4.

The particle size of the oxcarbazepine used in this example was estimated to be such that d(0.5) is of the order of 27 microns and not less than about 29% of this material would be retained on a 40 micron sieve. The following exemplifies how such estimation is reached. In order to evaluate the amount of oxcarbazepine that would be retained on a 40 micron screensieve analysis of large oxcarbazepine was preformed by "Fritsch" vibratory sieve shaker for wet sieving. The test was preformed with large oxcarbazepine characterized by d(0.5) of 16 microns. The result of the sieve analysis demonstrates that about 29% of the oxcarbazepine would be retained on a 40 micron screen. Therefore, it is obvious that sieving test of large oxcarbazepine used for example 8 and 9 will result by not less than 29% would be retained on 40 micron sieve.

### Example 9: Preparation of sprayed granulated oxcarbazepine formulation with extra-granular excipients.

The first two steps were as in example 8. In step 3 the oxcarbazepine sprayed granulate was mix together with extragranular excipients as follow:

| **Formulation of example 9 (Batch #13)** | **Amount/dose** |
|---|---|
| Oxcarbazepine sprayed granulate from step 2 [contains 600mg oxcarbazepine (after potency adjustment)] | 1659 mg |
| Lactose spray dried | 60 mg |
| Starch 1500 | 25 mg |
| Crosspovidone | 100 mg |
| Magnesium stearate | 10 mg |

In the fourth step The granulate mixture was subsequently pressed into tablets and a dissolution test was performed. The observed dissolution rate was similar to that for the TRILEPTAL® bio equivalent formulation as is shown in figure 4.

### Example 10: Preparation of sprayed granulated oxcarbazepine formulation.

In a first step 1 a preparation of a start dispersion from large Oxcarbazepine was prepared. 30g of Large Oxcarbazepine raw material (RM) [d(0.9)=248.5] was dispersed in a solution of 6g hypromellose (Pharmacoat 603) in 120g of purified water. The Pharmacoat was added to the water and mixed until clear mixture was obtained. Subsequently, the large Oxcarbazepine was added into the Pharmacoat water mixture and dispersed using a rotor stator (Brogtec) for 30 min at 9000rpm. The large Oxcarbazepine particle size distribution following rotor stator process, as measured by the laser diffraction method (Malvern Mastersizer S), was as follows:

| | d(0.9) | d(0.5) | d(0.9) |
|---|---|---|---|
| Large Oxcarbazepine | 2 microns | 14 microns | 59 microns |

In a second step the large Oxcarbazepine dispersion was used as spray granulation dispersion in fluid-bed-top-spray-granulation process. The granulate formulation contains the following substances:

| **Formulation of example 10 (Batch # 14)** | **Amount/dose** |
|---|---|
| Oxcarbazepine [as sprayed dispersion (from step 1)] | 600 mg |
| Pharmacoat [as sprayed dispersion (from step 1)] | 120 mg |
| Avicel PH 101 | 110 mg |
| Lactose monohydrate 100 mesh | 100 mg |
| Starch 1500 NF | 40 mg |
| Sodium starch glycolate | 50 mg |

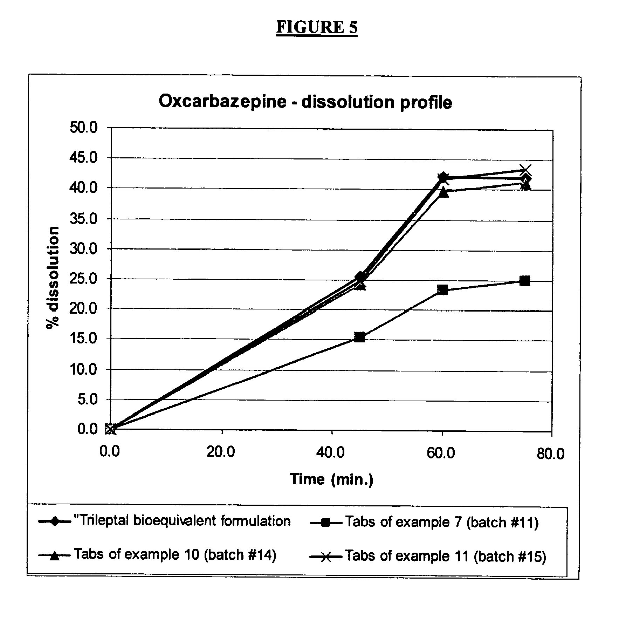

In the third step The granulate mixture was subsequently pressed into tablets and a dissolution test was performed. The observed dissolution rate was similar to that for the TRILEPTAL® bio equivalent formulation as is shown in figure 5.

### Example 11: Preparation of sprayed granulated oxcarbazepine formulation with extra-granular excipients.

The first two steps were as in example 10. In step 3 the oxcarbazepine sprayed granulate was mix together with extragranular excipients as follows:

| **Formulation of example 11 (Batch #15)** | **Amount/dose** |
|---|---|
| Oxcarbazepine sprayed granulate from step 2 [contains 600mg oxcarbazepine (after potency adjustment)] | 980 mg |
| Lactose spray dried | 120 mg |
| Starch 1500 | 50 mg |
| Magnesium stearate | 10 mg |

In the fourth step The granulate mixture was subsequently pressed into tablets and a dissolution test was performed. The observed dissolution rate was similar to that for the TRILEPTAL® bio equivalent formulation as is shown in figure 5.

For formulations prepared as per examples 8, 9, 10 & 11 where a single population of oxcarbazepine is used in a spray granulation process, the particle size of the oxcarbazepine used is preferably such that the d(0.5) value is between about 13 microns and about 30 microns

The following two examples exemplify spray granulated formulations having Oxcarbazepine having d(0.5) less than 2% and greater than 5% that would be retained on a 40 micron sieve.

### Example 12: Preparation of multi-modal oxcarbazepine formulation with a 20:80 ratio wherein a dispersion of small particles of oxcarbazepine is sprayed onto large oxcarbazepine particles in a spray granulation process.

In a first step 1 a preparation of a start dispersion from large Oxcarbazepine was prepared. 1500g of Large Oxcarbazepine raw material was dispersed in a solution of 300g hypromellose (Pharmacoat 603) in 6000g of purified water. The Pharmacoat was added to the water and mixed until a clear mixture was obtained. Subsequently, the large Oxcarbazepine was added into the Pharmacoat water mixture and dispersed using a rotor stator (Brogtec) for about 30 min. The large Oxcarbazepine particle size distribution, as measured by the laser diffraction method (Malvern Mastersizer S), was as follows:

| | d(0.1) | d(0.5) | d(0.9) |
|---|---|---|---|
| Large Oxcarbazepine | 25 | 68 | 312 |

In a second step 2 the particle size of the large oxcarbazepine was reduced by a high pressure homogenization process (MFIC microfluidizer M-1 10F). The particle size distribution of this final Oxcarbazepine (small) dispersion (small Oxcarbazepine), as measured by the laser diffraction method (Malvern Mastersizer S), was as follows:

| | d(0.1) | d(0.5) | d(0.9) |
|---|---|---|---|
| Small Oxcarbazepine | 0.3 | 0.9 | 8.3 |

In a third step 3 the small Oxcarbazepine final dispersion was used as spray granulation dispersion in fluid-bed-top-spray-granulation process and sprayed onto a mix of excipients and large oxcarbazepine. The large Oxcarbazepine particle size distribution, as measured by the laser diffraction method (Malvern Mastersizer S), was as mentioned in stage 1 of the current example. The granulate formulation contains the following substances:

| Batch # 16 | Amount/dose |
|---|---|
| Small Oxcarbazepine [as sprayed dispersion (from step 2)] | 480 mg |
| Pharmacoat (HPMC) [as sprayed dispersion (from step 2)] | 96 mg |
| Large Oxcarbazepine | 120 mg |
| Avicel PH 101 | 110mg |
| Lactose monohydrate 100 mesh | 100 mg |
| Starch 1500 NF | 40 mg |
| Crosspovidone | 50 mg |

In a fourth step this granulate was mixed together with extra-granular excipients as follows:

| **Formulation of example 12 (batch #17)** | **Amount/dose** |
|---|---|
| Oxcarbazepine granulate (batch #16) | 996 mg |
| Avicel PH 102 | 12 mg |
| Crosspovidone | 40 mg |
| Aerosil 200 | 4 mg |
| Magnesium stearate | 8 mg |

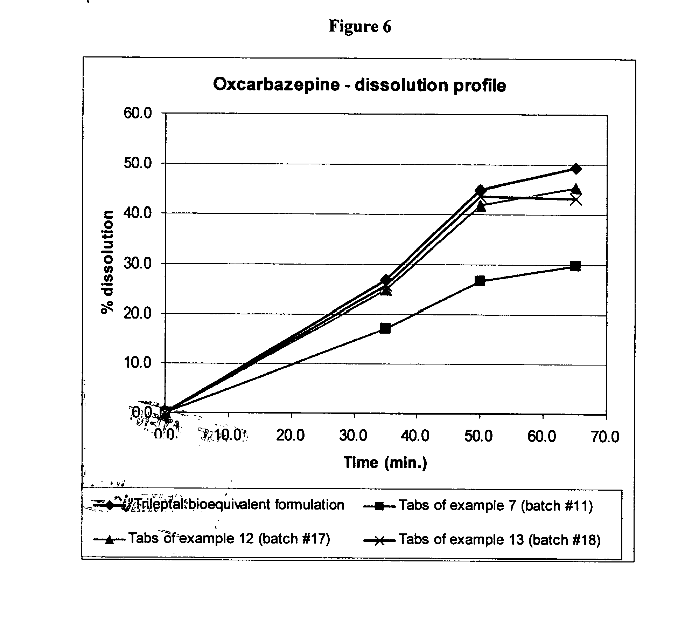

The granulate mixture was subsequently pressed into tablets and a dissolution test was performed. Additionally, the tablets were coated.
The observed dissolution rate was similar to that for the TRILEPTAL® bioequivalent formulation as is shown in figure 6. The particle size of the oxcarbazepine used in this example was estimated to be such that d(0.5) is of the order of 0.8 microns and about 12% of this material would be retained on a 40 micron sieve.
The evaluation of median particle size and sieve analysis is demonstrated in example 2.

### Example 13:

The first three steps were as in example 12. In step 4 the oxcarbazepine sprayed granulate was mix together with extra-granular excipients as follows:

| Formulation of example 13 (batch #18) | Amount/dose |
|---|---|
| Oxcarbazepine granulate (batch #16) | 996 mg |
| Lactose spray dried | 60 mg |
| Starch 1500 | 25 mg |
| Crosspovidone | 100 mg |
| Magnesium stearate | 10 mg |

The granulate mixture was subsequently pressed into tablets and a dissolution was performed. Additionally, the tablets were coated.

The observed dissolution rate was similar to that for the TRILEPTAL® bioequivalent formulation as is shown in figure 6. As the granulate is the same as was resultant from example 12 the particle size of the oxcarbazepine used in this example is similarly estimated to be such that d(0.5) is of the order of 0.8 microns and about 12% of this material would be retained on a 40 micron sieve.

### Example 14: Dissolution test

The tablets described in examples 1-13 were tested in a media containing a physiological surfactant and the procedure included gradual addition of volume and pH changes in order to simulate the gastrointestinal conditions. The dissolution procedure was carried out in an USP Apparatus II, paddle method, at 37°C and 50 rpm under the following conditions as in Table 2.

**Table 2: Dissolution procedure conditions.**

| **Time interval** | **Media** | **Volume** |
|---|---|---|
| 0-20 min | HCl 0.05N +NaCl 2g/l | 200 ml |
| 20-35 min | Lecithin 0.133%, Phosphate buffer, pH 6 | 600 ml |
| 35-65 min | Lecithin 0.16%, Phosphate buffer, pH 6 | 1000 ml |

The concentration of the dissolved oxcarbazepine was measured at the 35, 50, and 60 minute time periods. Due to the turbidity of lecithin solutions, the samples are preferably clarified before measurement by UV. To compare the dissolution profile with commercially available pharmaceutical compositions, a pharmaceutical composition (K-34926) which is bioequivalent to TRILEPTAL® was included in the dissolution experiments. Table 4 shows the observed dissolution profile for each of the tablets from examples 1-6, and 8-13, a TRILEPTAL® bioequivalent (K-34926) tablet, and a relatively slow dissolving pharmaceutical composition prepared according to the present invention (example 7).

The results shown are obtained in six separate experiments. In ***Figure 1*** is demonstrated the results of a dissolution test of the TRILEPTAL® bioequivalent composition (K-34926) and Examples 1 and 2 under the above described conditions. Similarly, in ***Figure 2*** the results are demonstrated of a dissolution test of the TRILEPTAL^{®} bioequivalent composition (K-34926), the relative slow composition (K-33529, example 7) and Examples 3 and 4. In ***Figure 3*** is demonstrated the results of a dissolution test of the TRILEPTAL^{®} bioequivalent composition (K-34926), the relative slow composition (K-33529; example 7), and Examples 5 and 6 under the same dissolution conditions. In ***Figure 4*** the results are demonstrated of a dissolution test of the TRILEPTAL® bioequivalent composition (K-34926), the relative slow composition (K-33529; example 7), and Examples 8 and 9 under the same dissolution conditions. In ***Figure 5*** is demonstrated the results of a dissolution test of the TRILEPTAL^{®} bioequivalent composition (K-34926), the relatively slow composition (K-33529; example 7), and Examples 10 and 11 under the same dissolution conditions. Finally, in ***Figure 6*** the results are demonstrated of a dissolution test of the TRILEPTAL^{®} bioequivalent composition (K-34926;), the relative slow composition (K-33529; example 7), and Examples 12 and 13 under the same dissolution conditions.

Consequently, the values in Table 3 for the TRILEPTAL® bioequivalent composition represent an average of the six experiments and the values in Table 3 for the relatively slow composition represent an average of four experiments. In Table 3 the dissolution profiles of various pharmaceutical compositions are shown in percent oxcarbazepine dissolved from the composition.

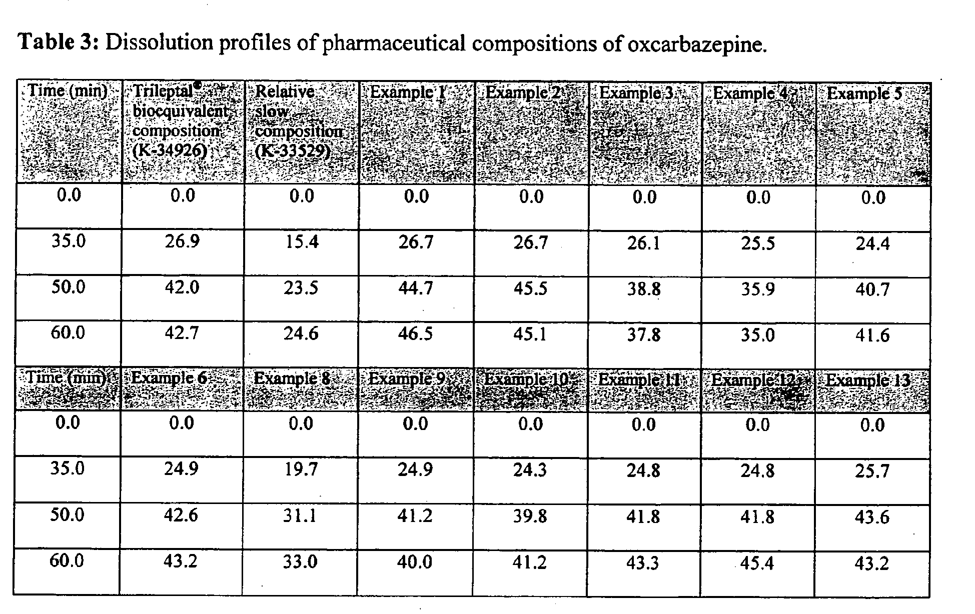

## Claims

1. A pharmaceutical composition comprising,
a) oxcarbazepine, and
b) at least one pharmaceutical excipient,
wherein the oxcarbazepine in the composition has a broad particle size distribution.

2. A pharmaceutical composition according to claim 1 wherein the oxcarbazepine in the composition has a broad particle size distribution wherein the difference in the value of d(0.5) and d(0.95) is greater than 38 microns, and the d(0.5) value is 35 microns or less.

3. A pharmaceutical composition according to claim 1 or claim 2 wherein the oxcarbazepine in the composition has a broad particle size distribution wherein the d(0.5) ranges from 12 to 35, or d(0.5) ranges from 0.01µ to 2.0 µ, and wherein in each case, the oxcarbazepine has a minimum residue on a 40 micron sieve of more than 5%.

4. The pharmaceutical composition according to any one of claims 1 to 3, wherein the broad particle size distribution comprises a multi-modal oxcarbazepine particle size distribution.

5. The pharmaceutical composition according to claim 4, wherein the multi-modal oxcarbazepine particle size distribution comprises at least one population of large oxcarbazepine particles selected from the group consisting of, a population of oxcarbazepine particles with a median size of at least 13 microns, a population of unground particles, particles that do not pass a 40 micron sieve, and mixtures thereof.

6. The pharmaceutical composition according to claim 5, wherein the median particle size of the at least one population of particles is at least about 40 microns.

7. The pharmaceutical composition according to claim 6, wherein the median particle size of the at least one population of particles is at least about 60 microns.

8. The pharmaceutical composition according to any one of claims 5 to 7, wherein the total amount of oxcarbazepine in the pharmaceutical composition comprises at least about 6% by weight of a population of large oxcarbazepine particles.

9. The pharmaceutical composition according to claim 8, wherein the total amount of oxcarbazepine in the pharmaceutical composition comprises at least about 10% by weight of a population of large oxcarbazepine particles.

10. The pharmaceutical composition according to claim 9, wherein the total amount of oxcarbazepine in the pharmaceutical composition comprises at least about 40% by weight of a population of large oxcarbazepine particles.

11. The pharmaceutical composition according to any one of claims 4 to 10, wherein the multi-modal oxcarbazepine particle size distribution comprises at least one population of small oxcarbazepine particles having a median particle size of less than about 6 microns.

12. The pharmaceutical composition according to claim 11, wherein the population of small oxcarbazepine particles has a median particle size less than about 3 microns.

13. The pharmaceutical composition according to claim 12, wherein the population of small oxcarbazepine particles has a median particle size less than about 2 microns.

14. The pharmaceutical composition according to any one of claims 11 to 13, wherein the total amount of oxcarbazepine in the pharmaceutical composition comprises at least about 5% of small oxcarbazepine particles.

15. The pharmaceutical composition according to claim 14, wherein the total amount of oxcarbazepine in the pharmaceutical composition comprises at least about 10% of small oxcarbazepine particles.

16. The pharmaceutical composition according to claim 15, wherein the total amount of oxcarbazepine in the pharmaceutical composition comprises at least about 20% of small oxcarbazepine particles.

17. The pharmaceutical composition of any one of claims 4 to 18, comprising at least one population of large oxcarbazepine particles and at least one population of small oxcarbazepine particles, wherein the total weight of oxcarbazepine in the pharmaceutical composition comprises from about 51% to about 95% by weight of large oxcarbazepine particles and from about 5% to about 49% by weight of small oxcarbazepine particles.

18. The pharmaceutical composition according to claim 17, wherein the population of small oxcarbazepine particles is from about 10% to about 45% by weight and the population of large oxcarbazepine particles from about 55% to about 90% by weight of the total weight of oxcarbazepine in the composition.

19. The pharmaceutical composition according to any one of claims 4 to 16, comprising at least one population of large oxcarbazepine particles and at least one population of small oxcarbazepine particles, wherein the total weight of oxcarbazepine in the pharmaceutical composition comprises from about 6% to about 40% by weight of large oxcarbazepine particles and from about 60% to about 94% by weight of small oxcarbazepine particles.

20. The pharmaceutical composition according to claim 19, wherein the population of small oxcarbazepine particles is from about 65% to about 90% by weight and the population of large oxcarbazepine particles from about 10% to about 35% by weight of the total weight of oxcarbazepine in the composition.

21. The pharmaceutical composition according to any one of claims 4 to 20, wherein the oxcarbazepine in the composition has at least two populations of different particle sizes wherein at least one population has small particle sizes and at least one population has large particle sizes, and wherein at least one of the populations of oxcarbazepine particles comprises spray granulated oxcarbazepine.

22. The pharmaceutical composition according to claim 21, wherein at least one population of large oxcarbazepine particles is spray granulated.

23. The pharmaceutical composition according to claim 21, wherein at least one population of small oxcarbazepine particles is spray granulated.

24. The pharmaceutical composition according to any one of claims 21 to 23, further comprising at least one excipient.

25. The pharmaceutical composition according to claim 24 wherein the at least one excipient is hypromellose.

26. The pharmaceutical composition according to any one of claims 1 to 25, wherein the oxcarbazepine has a median particle size of not more than 2 microns and has a minimum residue on a 40 micron sieve of more than 5%.

27. The pharmaceutical composition according to any one of claims 1 to 25, wherein the oxcarbazepine has a median particle size of not less than 13 microns and has a minimum residue on a 40 micron sieve of more than 5%.

28. The pharmaceutical composition according to any one of claims 1 to 25, wherein the oxcarbazepine has a median particle size of 2 to 12 microns and has a minimum residue on a 40 micron sieve of more than 5%.

29. The pharmaceutical composition according to any one of the preceding claims, wherein the composition is **characterized by** a rate of dissolution such that greater than 20% of the oxcarbazepine is dissolved in 35 minutes and between about 30% and about 50% is dissolved in about 50 minutes.

30. The pharmaceutical composition according to claim 29, wherein 40% or more of the oxcarbazepine is dissolved in about 50 minutes.

31. The pharmaceutical composition according to any one of claims 1 to 28, having a dissolution profile in an aqueous buffer simulating the gastrointestinal environment of
a) no more than about 30% of the total amount of oxcarbazepine is dissolved from the composition after 35 minutes of measurement in a dissolution apparatus;
b) greater than 20% of the total amount of oxcarbazepine is dissolved from the composition in 35 minutes of measurement in a dissolution apparatus;
c) from about 30% to about 50% of the total amount of oxcarbazepine is dissolved from the composition after 50 minutes of measurement in a dissolution apparatus; and
d) from about 30% to about 50% of the total amount of oxcarbazepine is dissolved from the composition after 60 minutes of measurement in a dissolution apparatus.

32. The pharmaceutical composition according to claim 31, wherein 40% or more of the total amount of oxcarbazepine is dissolved from the composition after 50 minutes.

33. The pharmaceutical composition according to claim 31 or claim 32, wherein the aqueous buffer simulating the gastrointestinal environment comprises;
a) 0.05 N HCI and 2g/l NaCl for the time interval from 0 to 20 minutes of dissolution;
b) 0.133% lecithin in a phosphate buffer at pH 6 for the time interval from 20 to 35 minutes of dissolution; and
c) 0.16% lecithin in a phosphate buffer at pH 6 for the time interval from 35 to 65 minutes of dissolution.

34. The pharmaceutical composition according to any one of claims 1 to 28, having a dissolution profile in media simulating the gastrointestinal environment, wherein at least 30% of the total amount of oxcarbazepine is dissolved from the composition within 60 minutes.

35. The pharmaceutical composition according to claim 34, having a dissolution profile in media simulating the gastrointestinal environment wherein at least 40% of the total amount of oxcarbazepine is dissolved from the composition within 60 minutes.

36. The pharmaceutical composition according to any one of claims 1 to 28, having a dissolution profile in media simulating the gastrointestinal environment, wherein at least 30% of the total amount of oxcarbazepine is dissolved from the composition within 50 minutes.

37. The pharmaceutical composition according to any one of claims 1 to 28, having a dissolution profile in media simulating the gastrointestinal environment, wherein at least 30% of the total amount of oxcarbazepine is dissolved from the composition within 35 minutes.

38. A pharmaceutical composition comprising,
a) spray-granulated oxcarbazepine; and
b) at least one pharmaceutical excipient.

39. The pharmaceutical composition according to claim 38, wherein the oxcarbazepine comprises at least one population of large oxcarbazepine particles selected from the group consisting of a population of oxcarbazepine particles having d(0.1) of about 21 microns, d(0.5) of about 71 microns and d(0.9) of about 248 microns, a population of oxcarbazepine having been subjected to minimal grinding, a population of unground oxcarbazepine, a population of oxcarbazepine having particles with a median size greater than 13 microns, and a population of oxcarbazepine particles that do not pass a 40 micron sieve, and mixtures thereof.

40. A method for preparing a pharmaceutical composition comprising oxcarbazepine as defined in any one of claims 1 to 37 comprising the steps of:
a) providing oxcarbazepine having a broad particle size distribution;
b) providing at least one excipient;
c) combining the oxcarbazepine with the at least one excipient.

41. A method for preparing a pharmaceutical composition comprising oxcarbazepine having a multi-modal oxcarbazepine particle size distribution comprising the steps of;
a) providing oxcarbazepine which comprises two or more populations of oxcarbazepine particles of different particle size distributions;
b) providing at least one excipient;
c) forming at least one granulate comprising at least one of said oxcarbazepine populations; and
d) combining the granulated oxcarbazepine with any remaining ungranulated oxcarbazepine and at least one excipient to form a final granulate blend.

42. The method according to claim 41, wherein at least one of the two to more populations of oxcarbazepine particles of different particle size distribution is granulated separately.

43. The method according to claim 41 or claim 42, wherein the two or more populations of oxcarbazepine particles of different particle size distributions are provided by milling a portion of the total amount of oxcarbazepine forming a population of small oxcarbazepine particles.

44. The method according to claim 43, further comprising de-agglomerating the portion of the total amount of oxcarbazepine.

45. The method according to claim 43 or claim 44, wherein milling is carried out in a liquid dispersion by a high pressure homogenizer.

46. The method according to any one of claims 43 to 45, wherein the remaining portion of the total amount of oxcarbazepine is only slightly milled.

47. The method according to any one of claims 43 to 46, wherein milling is carried out by a milling process selected from the group consisting of using an air jet mill with low grinding pressure and using an homogenizer at a low rotation rate in a liquid dispersion.

48. The method according to any one of claims 41 to 47, further comprising the steps of
d) optionally mixing the final granulate blend with one or more excipients to form a tableting mixture; and
e) pressing either the tableting mixture or the final granulate blend into tablets; and optionally
f) coating the tablets.

49. The method according to any one of claims 41 to 48, wherein said at least one granulate is formed by a spray granulation process.

50. The method according to claim 49, wherein said spray-granulation process comprises the steps of:
i) preparing a dispersion of at least one population of oxcarbazepine particles forming at least one oxcarbazepine dispersion;
ii) spraying the oxcarbazepine dispersion onto one or more excipients forming at least one spray granulate.

51. The method according to claim 49 or claim 50, wherein spray granulation is carried out in a fluidized bed equipment.

52. The method according to any one of claims 49 to 51, wherein the spray granulation process is carried out by using a spraying method selected from the group consisting of top spray, bottom spray, and tangential/powder spray.

53. The method of any one of claims 50 to 52, wherein the oxcarbazepine dispersion is sprayed onto one or more excipients and at least one of the remaining populations of oxcarbazepine forming an oxcarbazepine spray granulate which contains at least two populations.

54. The method of any one of claims 40 to 53 wherein the oxcarbazepine particles have a median particle size of less than 2 microns and greater than 5% of the oxcarbazepine particles would be retained on a 40 micron screen.

55. A pharmaceutical composition comprising oxcarbazepine as defined in any one of claims 1 to 39 for use in therapy.

56. A pharmaceutical composition comprising oxcarbazepine as defined in any one of claims 1 to 39 for use in treating epileptic seizures or Parkinson's disease.

57. The use of oxcarbazepine for the manufacture of a pharmaceutical composition as defined in any one of claims 1 to 39 for treatment of epileptic seizures or Parkinson's disease or neuropathic pain.
